(19) **Europäisches Patentamt European Patent Office Office européen des brevets**

(11) **EP 2 819 571 B1**

(12) **EUROPEAN PATENT SPECIFICATION**

(45) Date of publication and mention of the grant of the patent:
**16.09.2020 Bulletin 2020/38**

(21) Application number: **13719607.7**

(22) Date of filing: **25.02.2013**

(51) Int Cl.:
***A61B 5/00*** *(2006.01)*     ***A61B 5/11*** *(2006.01)*

(86) International application number:
**PCT/IB2013/051509**

(87) International publication number:
**WO 2013/128364 (06.09.2013 Gazette 2013/36)**

(54) **PROCESSING A SIGNAL REPRESENTING A PHYSIOLOGICAL RHYTHM**

VERARBEITUNG EINES EINEN PHYSIOLOGISCHEN RHYTHMUS DARSTELLENDEN SIGNALS

TRAITEMENT D'UN SIGNAL REPRÉSENTANT UN RYTHME PHYSIOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.03.2012 US 201261605355 P**

(43) Date of publication of application:
**07.01.2015 Bulletin 2015/02**

(73) Proprietors:
• **Koninklijke Philips N.V.**
**5656 AG Eindhoven (NL)**
Designated Contracting States:
**AL AT BE BG CH CY CZ DK EE ES FI FR GB GR HR HU IE IS IT LI LT LU LV MC MK MT NL NO PL PT RO RS SE SI SK SM TR**
• **Philips GmbH**
**22335 Hamburg (DE)**
Designated Contracting States:
**DE**

(72) Inventors:
• **BRÜSER, Christoph**
**Eindhoven 5656 AE (NL)**
• **LEONHARDT, Klaus, Steffen**
**Eindhoven 5656 AE (NL)**

(74) Representative: **Philips Intellectual Property & Standards**
**High Tech Campus 5**
**5656 AE Eindhoven (NL)**

(56) References cited:
**WO-A1-2011/013048     US-A1- 2007 276 202
US-A1- 2010 249 628     US-A1- 2011 251 502**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

**Description**

TECHNICAL FIELD OF THE INVENTION

This invention relates to a method of processing a signal representing a physiological rhythm of a subject. In one embodiment, the invention provides a continuous local interval estimation thereby delivering a method for robust periodicity computation in bio-signals.

BACKGROUND TO THE INVENTION

Accurate knowledge of physiological rhythms, such as heart rate or respiratory rate, is of crucial importance in many healthcare applications including diagnosis, patient monitoring, and treatment. Accordingly, the estimation of the time-varying frequencies of these rhythms from conventional clinical measurement modalities such as electrocardiography (ECG), pulse oximetry, or respiratory inductance plethysmography is a well-studied problem. While for some applications, it might be sufficient to estimate only an average frequency over multiple periods of the underlying rhythm, others such as heart rate variability (HRV) analysis require an event-to-event resolution.

Due to demographic changes and the growing numbers of patients with chronic conditions, home monitoring of a patient's health status will play an important role in future healthcare systems. Recent studies have shown that home telehealth approaches can lead to a significant reduction of hospital admissions and numbers of bed days of care.

Since conventional measurement modalities are rarely applicable outside of clinical settings, unobtrusive and unconstrained measurement systems are currently emerging as a way to allow continuous long-term monitoring of a patient's health status. These systems aim to provide two advantages over current approaches in that they usually require no user interaction or compliance to perform their measurements and they do not interfere with the user's daily routine. Unobtrusive measurement systems based on a variety of different principles are known. These include capacitively coupled electrodes for ECG measurements in a chair or a car seat, capacitive respiration measurement in bed, radar, millimeter-wave interferometry, and ballistocardiography (BCG) or seismocardiography (SCG) sensors integrated into beds, chairs and weighing scales.

While these approaches offer the aforementioned advantages over established methods, they also come with their own set of drawbacks. Most notably, the signal quality can be highly varying and unreliable due to the often uncontrolled environment in which the measurements are performed. For some types of sensors, the morphology of the recorded signals can change drastically depending on the orientation and position of the user with respect to the sensor. This can be observed, for instance, in the case of bed-mounted BCG sensors which record cardiac vibrations caused by the contraction of the hearts and the ejection of blood into the aorta.

Under these circumstances, reliable estimation of instantaneous frequencies (or their inverse: local interval lengths) can pose a significant challenge to algorithms which were originally developed for clinical use. These algorithms are often based on detecting a particular feature of the signal relating to the event of interest, such as the QRS complex in the ECG. Instantaneous frequencies are then computed by differencing successive occurrence times of these events. In the case of the ECG, sophisticated methods have been proposed to improve the robustness of the instantaneous heart rate estimation. However, these methods are usually limited to one type of signal and require extensive prior knowledge about the expected morphology of the waveform. As mentioned above, these requirements do not hold for unobtrusive sensors types.

US 2011/025102 A1 discloses a method for analyzing a ballistocardiogram signal to determine a heart rate, the method comprising determining initial time estimate for a first heart beat in the ballistocardiogram signal, computing iteratively estimates for subsequent heart beats in the ballistocardiogram signal using the initial time estimate, wherein each iteration in a step of computing comprises evaluating a target function that comprises a weighted sum of scoring functions, and wherein each iterative step of computing estimates for subsequent heart beats in a ballistocardiogram signal is limited to a target interval after the time estimate found in the previous iterative step of computing.

SUMMARY OF THE INVENTION

It is therefore an object of the invention to improve upon the known art.

According to the present invention, there is provided a method of processing a signal representing a physiological rhythm of a subject as defined in claim 1.

Owing to the invention, it is possible to provide a robust algorithm of continuous local interval estimation for the estimation of interval lengths or frequencies which does not require any prior knowledge about the morphology of the analyzed signal. With just minor adjustments to a few basic parameters, the method can be adapted to accurately extract instantaneous frequencies from different physiological rhythms such as heart rate or respiratory rate, even if they coexist in the same signal.

While the invention can be used to extract instantaneous frequencies from a single physiological signal, it is also capable of processing multiple synchronous signals representing the same physiological rhythm. Such signals could, for instance, be recorded by an array of unobtrusive sensors, such as a matrix of bed-mounted BCG sensors. By performing a plurality of interval length estimation methods on the filtered signals in the analysis window, and determining an interval length from the sum of the outputs of the plurality of interval length estimation methods over all signals, the invention exploits the redundancy present in multiple signals and thus improves the interval estimation accuracy and reliability.

Reliable and accurate estimation of instantaneous frequencies of physiological rhythms, such as heart rate or respiratory rate, is therefore provided for many healthcare applications. Robust estimation is especially challenging when unobtrusive sensors are used for continuous health monitoring in uncontrolled environments, because these sensors can create significant amounts of potentially unreliable data. The invention therefore provides a flexible method for the robust estimation of local (event-to-event) intervals from these signals. The method does not require any prior knowledge about the morphology of the analyzed waveforms and can thus be easily applied to a variety of different signals and measurement modalities.

Two applications of the algorithm to beat-to-beat heart rate and breath-to-breath respiratory rate estimation have been validated using a conventional as well as an unobtrusive sensor signal. An evaluation data set, containing over 212000 heart beats and 52000 breaths, has been used to validate the method. When applied to signals recorded by an unobtrusive bed sensor, the improved method achieved a mean beat-to-beat heart rate error of 0.86 beats/min with a mean coverage of 90.59%. Using the same signals to estimate breath-to-breath respiratory rate resulted in a mean error of 0.30 breaths/min and a coverage of 89.86%.

The overlap of the first analysis window and the second analysis window should be greater than 50% of the size of the first and/or second analysis windows. The use of closely overlapping analysis windows increases the robustness of the method, as an interval will be present in multiple analysis windows meaning that there is a much increased likelihood of correctly calculating the interval length in the filtered signal.

Advantageously, the method further comprises calculating a mean interval length for multiple analysis windows, generating upper and lower threshold limits for the interval length from the calculated mean interval length and a predetermined delta and discarding any determined interval lengths that are outside the generated upper and lower threshold limits. The creation of upper and lower threshold limits for the interval length will create a bounding of acceptable calculated interval lengths. This will mean that any calculated interval lengths that are outside the thresholds will be discarded as they are likely to be false measures or the result of distortion or artifacts within the signal.

Ideally, the method further comprises accessing weighting factors for the plurality of interval length estimation methods and wherein the step of summing the outputs of the plurality of interval length estimation methods comprises summing the outputs of the plurality of interval length estimation methods according to the accessed weighting factors. The method can be adjusted by the use of weighting factors for the different interval length estimation methods. These weighting factors may be specific to individual applications of the process, for example, in order to provide the best results for the specific application. The use of the weighting factors increases the flexibility and accuracy of the method.

The method further comprises detecting a peak in the filtered signal in consecutive analysis windows corresponding to an end of the determined interval length and calculating a median determined interval length from the set of matching peaks of the same amplitude. The method of calculating the interval length within the signal can be further refined by comparing intervals in consecutive analysis windows. Since the windows overlap significantly, it can be assumed that the same interval will be present in different analysis windows. If the analysis windows overlap by 90%, for example, then up to five consecutive analysis windows can be used. A peak is detected that will correspond to one end of the interval within each analysis window. If the amplitude of these peaks is the same, then it can be assumed that the same point in the signal is being looked at, and the median of the determined interval lengths can be selected as an output as a more accurate figure than an interval length that has not been further processed in this manner.

BRIEF DESCRIPTION OF THE DRAWINGS

Embodiments of the present invention will now be described, by way of example only, with reference to the accompanying drawings, in which:-

Fig. 1 is a schematic diagram of a subject being monitored while in bed,
Fig. 2 is a graph of a subject's heart rate (lower chart) and the detected intervals in the subject's heart rate (upper chart),
Fig. 3 is a graph of a subject's heart rate (upper chart) and the detected intervals in the subject's heart rate using different techniques (lower charts),
Fig. 4 is a graph of four different analysis windows taken from a signal,
Fig. 5 is a graph of a comparison of three different interval length estimation techniques,
Figs. 6 and 7 are graphs comparing two different interval length estimation algorithms for two different ECG signals,

Fig. 8 is a graph comparing error occurrence in two different interval length estimation algorithms for different signal to noise ratios in various ECG signals,

Fig. 9 is a graph showing the effect of a threshold on coverage against error of an interval length estimation algorithm,

Figs. 10 and 11 are graphs of modified Bland-Altman plots of the local interval errors in different signals,

Fig. 12 is a schematic diagram of a subject being monitored using two sensors, and

Fig. 13 is a flowchart of a method of processing a signal representing a physiological rhythm of a subject.

## DETAILED DESCRIPTION OF PREFERRED EMBODIMENTS

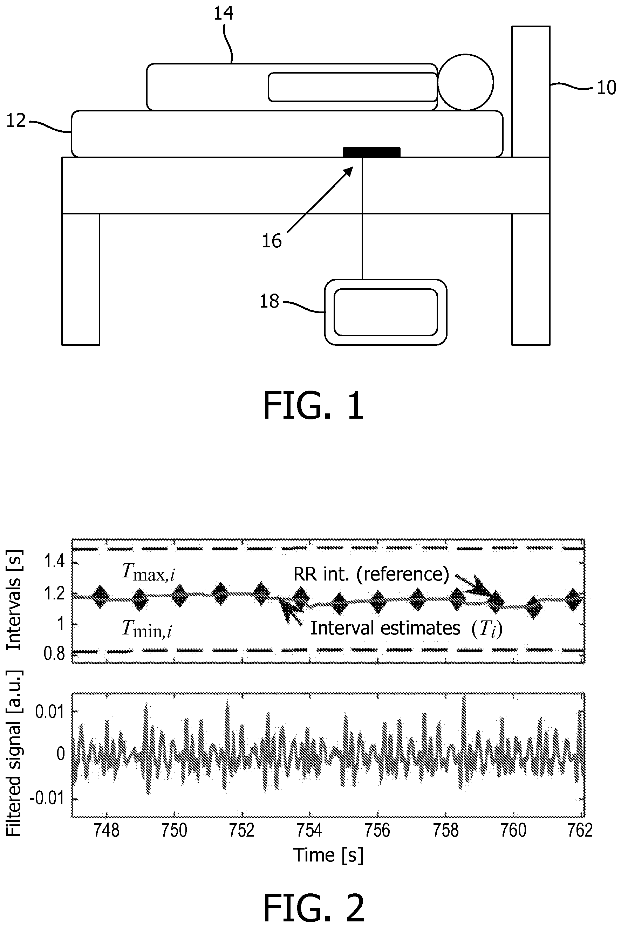

Figure 1 illustrates a bed 10 with a mattress 12 on which a subject 14 being monitored will sleep at night. A sensor 16 is placed between the bed 10 and mattress 12 and will generate a signal that represents a physiological rhythm of the subject. This rhythm could be the subject's heartbeat or the user's breath frequency, for example. The sensor 16 is connected to a local processing unit 18 which can perform analysis of the received signal in order to perform constant monitoring of the health of the subject 14. The sensor 16 can be connected to remote systems that can provide further processing and or monitoring functions. The purpose of the system shown in Figure 1 is to provide continual monitoring of the health of the subject 14, in such a way that the subject 14 is not required to wear any sensors on their body nor has their natural sleep disturbed in any way. Constant monitoring is provided by the sensor 16, which may be monitoring multiple different physiological parameters of the subject 14. A continuous signal is provided from the sensor 16 that is processed by the device 18 for monitoring the health of the subject 14. These systems allow subjects to monitored in their own home, without the need for expensive hospital monitoring of the subject 14.

The nature of the sensor 16 is such that, because it is not directly connected to the subject's body in a defined medical environment, it does not provide a signal that has the same level of reliability as does one that is measured in a controlled environment using medical professionals. The subject's movement while they are in the bed 10 and other environmental factors will result in a signal that has a high level of noise and artifacts that can make difficult the accurate measurement of the subject's physiological parameters. Since the subject's health is being monitored by a system such as that shown in Figure 1, the accuracy of the output of the processing device 18 is very important.

The processing device 18 runs an algorithm that provides a general framework for the estimation of local (event-to-event) interval lengths from the signals received from the sensor 16 containing time-varying physiological rhythms. Given a signal x(t) then $t_k$, k ε {1, ... , N} denotes the times at which particular events of interest (such as heart beats or breaths) occur in x(t). The local interval lengths as well as the local frequency can then be defined as:

$$T_k \;=\; t_k - t_{k-1} \qquad \text{and} \tag{1}$$

$$f_k \;=\; \frac{1}{T_k}, \tag{2}$$

respectively.

Unlike many existing methods for determining the time-varying frequency of a non-stationary signal, this method does not rely on fiducial markers to detect $t_k$ and then compute $T_k$ nor does it apply classical time-frequency analysis using long windows covering multiple intervals. Instead, local periodicity is estimated using a short adaptive analysis window (ideally containing two events of interest). This window is shifted across the signal using increments that are short with respect to the expected interval lengths, thus causing each interval to appear in multiple consecutive analysis windows. This method gains its robustness by exploiting this redundancy as well as by combining multiple methods to estimate the periodicity in each analysis window.

First the signal is pre-processed. The expression $x_{raw}[n]$ denotes the raw digital sensor signal with a sampling frequency of $f_s$. At first, $X_{raw}$ is pre-processed using a band-pass filter that is appropriate for the physiological rhythm which it is desired to extract from the received signal. The filtered signal is denoted as x[n]. In the case of the BCG signals used for demonstration, there is applied an equiripple finite impulse response (FIR) filter with cut-off frequencies of 0.5 and 30 to look for the subject's heart rate. The filter should be designed such that the base frequencies as well as the higher-order harmonics of the rhythm under analysis are located in the passband of the filter.

Figure 2 shows, in the lower half of the Figure, a filtered signal in arbitrary units (a.u.) that has been derived from a subject's BCG signal. The upper half of the Figure shows continuous interval estimates ($T_i$), measured in seconds, of the algorithm from the subject's BCG signal. The locations of diamond markers indicate the occurrence of R peaks as well as the corresponding RR interval length in a simultaneously recorded reference ECG. The boundaries of admissible interval lengths ($T_{min,i}$ and $T_{max,i}$) are shown as the dashed lines.

The improved method iteratively shifts an adaptive analysis window across the signal. During each iteration, the local interval length $T_i$ in the analysis window is estimated. In order to improve the robustness of the estimation, the admissible values of $T_i$ are constrained by two adaptive thresholds $T_{min,i}$ and $T_{max,i}$. Figure 2 shows the continuous interval estimates and thresholds obtained from a sample BCG signal. The i-th iteration of the algorithm consists of the following steps:

1. Update admissible interval lengths based on the mean of the previously estimated interval lengths $T_{mean,i}$ and the search window width $2\Delta T$.

$$T_{\min,i} \quad = \quad T_{\mathrm{mean},i} - \Delta T \tag{3}$$

$$T_{\max,i} \quad = \quad T_{\mathrm{mean},i} + \Delta T \tag{4}$$

2. Extract an analysis window $w_i[v]$ centred at $n_i$.

$$w_i[\nu] = x[n_i + \nu], \quad \nu \in \{-T_{\max,i} \cdot f_s, \ldots, T_{\max,i} \cdot f_s\} \tag{5}$$

3. Compute maximum peak-to-peak amplitude of the analysis window.

$$r_i = \max_\nu(w_i[\nu]) - \min_\nu(w_i[\nu]) \tag{6}$$

4. If $r_i$ is within specific thresholds

$$\alpha R_i < r_i < R_i, \quad \alpha \in [0,1), \tag{7}$$

the presence of a valid signal amplitude in the analysis window is assumed. Otherwise, the window is marked as invalid (artifact) and the algorithm skips to step 7.
5. Estimate local interval length $T_i$ in $w_i[v]$, described in more detail below.
6. Update mean of estimated intervals and amplitude (artifact) threshold for the next iteration.

$$T_{\mathrm{mean},i+1} = (1 - \beta)T_{\mathrm{mean},i} + \beta T_i, \quad \beta \in [0,1] \tag{8}$$

$$R_{i+1} = (1 - \gamma)R_i + \gamma \kappa r_i, \quad \gamma \in [0,1], \quad \kappa > 1 \tag{9}$$

7. Shift the centre of the analysis window for the next iteration by a constant value $\Delta T$.

$$n_{i+1} = n_i + \Delta t \cdot f_s \tag{10}$$

Step 5 of the method above comprises the interval length estimation, which is now described in more detail. For each analysis window $w;[v]$, the local interval length $T_i$ is estimated. A variety of established methods can be applied to determine the most likely local interval lengths, such as auto-correlation or spectral analysis. In the preferred embodiment, three such methods are chosen to combine which are evaluated at each admissible discrete interval length $N \in \{N_{min,i}, \ldots, N_{max,i}\}$, with $N_{min,i} = T_{min,i} \cdot f_s$ and $N_{max,i} = T_{max,i} \cdot f_s$. For better readability, the iteration index i is omitted in the following description.

1. The first method used is a modified P-spectrum. The modified P-spectrum computes a measure of similarity for all discrete lags, i.e. interval lengths N, which are within the search boundaries. For each lag N, the analysis window is configured into a matrix:

$$\mathbf{A}_N = \left( \begin{array}{cccc} w[0] & w[1] & \ldots & w[N_{\max,i}] \\ w[-N] & w[1-N] & \ldots & w[N_{\max,i}-N] \end{array} \right).$$ (11)

Singular value decomposition of $A_N$ results in $\mathbf{A}_N = \mathbf{U}_N \mathbf{S}_N \mathbf{V}_N^T,$ where $S_N = \mathrm{diag}(s_{N,1}, s_{N,2})$. If the signal in the analysis window is strictly periodic with a period of N, both rows of $A_N$ are identical and the matrix has a rank of 1 with $s_{N,1} > 0$ and $s_{N,2} = 0$ (i.e. $s_{N,1}/s_{N,2} \to \infty$). In the case of a nearly periodic signal, $A_N$ can be a full rank matrix. However, $s_{N,1}$ will still be significantly greater than $s_{N,2}$. Hence, the quotient $s_{N,1}/s_{N,2}$ is chosen as a measure of the linear dependence between the two matrix rows. This measure is always positive and does not differentiate between positive and negative dependence. Therefore there is also included the signs of the amplitude scaling factors $u_{N,1} \cdot s_1$ in the definition of the modified P-spectrum:

$$S_{\mathrm{P}}[N] = \mathrm{sgn}(u_{N,11})\,\mathrm{sgn}(u_{N,12})\frac{s_{N,1}}{s_{N,2}}.$$ (12)

This results in positive values for $S_P[N]$ when the scaling factors have identical signs and both signal segments are positively related. As such, the P-spectrum is a time-domain method similar to auto-correlation. Note that w[0] denotes the centre of the analysis window. The limits of the P-spectrum, as well as the following methods, are chosen under the assumption that the two events that form the boundaries of the interval to be estimated are located to the left and to the right of w[0], respectively. 2. The second method used is a maximum spectrum analysis. The maximum spectrum is based on the amplitude information of the signal. It is defined as:

$$S_{\mathrm{max}}[N] = \max_{\nu \in \{0,\ldots,N\}} (w[\nu] + w[\nu - N]).$$ (13)

For each possible lag N, the maximum amplitude of any pair of samples which are exactly N sampling intervals apart is determined. If two peaks exist in the analysis window which are N samples apart, $S_{\mathrm{max}}[N]$ will possess a maximum at that value of N. 3. The third method used is a cepstrum analysis. The power cepstrum C {.} of the analysis window is defined as the power spectrum of the logarithm of the power spectrum of w[v]:

$$\mathcal{C}\{w[\nu]\} = \left| \mathcal{F}\{\log(|\mathcal{F}\{w[\nu]\}|^2)\} \right|^2.$$ (14)

The log-power spectrum of a periodic signal (which is not a perfect sinusoid) consists of peaks at the fundamental frequency of the signal and its harmonics. These peaks are periodic with a period length corresponding to the fundamental frequency. Hence, a peak corresponding to the period of the underlying signal appears in the derived cepstrum. Analogous to the other methods, the cepstrum is evaluated at all interval lengths N which are within the search limits:

$$S_{\mathrm{C}}[N] = \mathcal{C}\{w[\nu]\}[N].$$ (15)

Each of the resulting three functions takes larger values for more likely local interval lengths N. In order to combine the three functions, they are scaled to a range of [0,1] resulting in the normalized functions $S'_{\mathrm{P}}$, $S'_{\mathrm{max}}$, and $S'_{\mathrm{C}}$. The scaled functions are then weighed and summed up to produce a combined indicator of the local interval length:

$$S_{\mathrm{sum}}[N] = m_1 S'_{\mathrm{P}}[N] + m_2 S'_{\mathrm{max}}[N] + m_3 S'_{\mathrm{C}}[N].$$ (16)

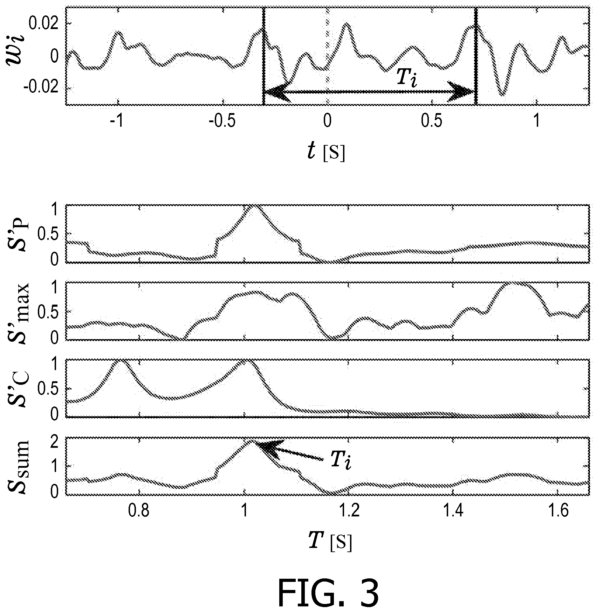

The weight vector m = $(m_1\ m_2\ m_3)^T$ was determined experimentally based on example applications, which is discussed in more detail below. As shown in Figure 3, the local interval length $T_i$ in the analysis window is then estimated by finding the discrete interval length $N_i$ corresponding to the maximum of $S_{sum}[N]$:

$$N_i = \arg\max_N S_{sum}[N] \qquad (17)$$

$$T_i = \frac{N_i}{f_s}. \qquad (18)$$

Figure 3 shows, from top to bottom, the analysis window ($w_i$) from a BCG signal and the normalized outputs of the three interval estimation methods discussed here: P-spectrum $\left(S'_P\right)$, maximum spectrum $\left(S'_{max}\right)$, and cepstrum $\left(S'_C\right)$ as well as their sum ($S_{sum}$). The analysis window ($w_i$) is measured in seconds as t[s] with 0 indicating the centre of the analysis window. The three interval estimation methods produce graphs that indicate the likely length of the interval $T_i$ within the analysis window measured in seconds as T[s]. The first method, P-spectrum $\left(S'_P\right)$, produces a clear peak at just larger than 1 second. The other two methods provide more indefinite results with peaks in more than one location. However, the sum of the indicator functions shows a clear peak at the fundamental interval length found in the analysis window. This is the interval $T_i$ and one such interval is shown on the analysis window of this Figure, for illustration purposes.

The use of three methods for interval estimation might, at first glance, appear redundant. However, the three selected methods complement one another as Figure 3 shows. In this example, both cepstrum and max-spectrum, have their highest peaks at different interval lengths which would result in a large estimation error if taken individually. When combined with the P-spectrum, however, the overall estimate is accurate. While the P-spectrum appears to provide the clearest estimate in this example, this is not always the case. Therefore, a joint consideration of all three methods is beneficial.

Those skilled in the art will appreciate that the described method can also be used to process a set of multiple synchronously recorded signals instead of a single one as shown in this example. For this purpose, a concomitant analysis window is extracted from each signal under analysis, and the plurality of interval estimation methods is applied to analysis window of each signal. The sum in equation (16) and the weight vector m are then extended to consider the entire set of interval estimators applied to all analysis windows. Thus, all available signals can be advantageously fused to obtain a more robust and accurate interval estimate.

The improved algorithm described above can be extended. In its basic form, the algorithm produces multiple estimates for each local interval. This redundancy can be exploited in order to provide more robust results by merging all estimates belonging to the same underlying interval. To this end, it is possible to use an extended version of the basic algorithm.

In the extended version, for each analysis window, after computing $T_i$, as described above, there is then located a distinctive landmark feature related to the estimated interval. Each estimate of an individual interval should yield the same distinctive feature which can then be used to group these estimates together. The largest peak in the signal is used to form the right-hand boundary of the interval as the landmark feature. Let $M_i$ denote the set of peaks located in the right half of the analysis window $w_i[v]$. The global location of the right boundary of the interval $T_i$ is defined as:

$$P_i = n_i + \arg\max_{m \in M_i} \left( w_i[m] + w_i[m - N_i] \right). \qquad (19)$$

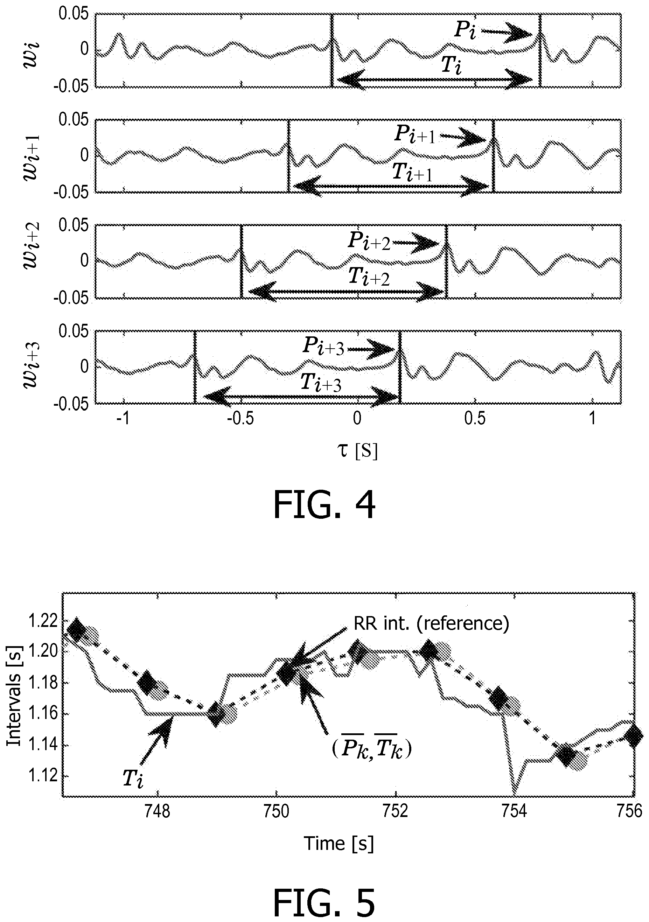

Figure 4 shows the analysis windows of multiple consecutive iterations and the derived interval lengths $T_i$ and peak locations $P_i$. The same interval appears in all analysis windows and similar values for $T_i$ lead to identical $P_i$ values for each analysis window. Thus, these estimates can easily be identified as belonging to the same interval. It should be stressed that the peak locations $P_i$ are solely used to group interval estimates together. Hence, their exact location is not important for the task of accurately estimating local intervals. Figure 4 shows the analysis windows of four consecutive algorithm iterations from a BCG signal. The same local interval is analyzed producing similar interval estimates ($T_i$). For each estimate, the same right boundary peak ($P_i$) is identified.

Let $\overline{P_k}$ denote the k-th unique value among all values of $P_i$. It is then possible to define the set of interval estimates belonging to that values of $\overline{P_k}$ as:

$$\mathbf{T}_k = \{T_i \mid P_i = \overline{P_k}\}. \tag{20}$$

The median of this set is computed to obtain a robust estimate of the local interval length:

$$\overline{T_k} = \mathrm{median}(\mathbf{T}_k). \tag{21}$$

Thus, the final output of the algorithm consists of pairs $\overline{P_k}$, $\overline{T_k}$) of peak locations and corresponding interval length estimates. Figure 5 shows an example of the extended algorithm output and how the combination of multiple interval estimates compensates outliers. In Figure 5 the output of the extended algorithm obtained from the same BCG signal as Figure 2 is the solid line $T_i$. The grey dots indicate the peak locations and corresponding interval length estimates ($\overline{P_k}$, $\overline{T_k}$) computed by the algorithm and the black diamonds indicate the reference values. By merging individual estimates ($T_i$), outliers can be eliminated so that the final output correlates highly with the simultaneously recorded reference RR intervals.

The set of estimates $T_k$ can also be used to formulate quality heuristics which can be applied to assess the confidence in the final interval estimate $\overline{T_k}$. For instance, the standard deviation of $T_k$ can be computed to quantify how well the estimates agree, according to the following formula:

$$Q_{\mathrm{std},k} = \frac{\mathrm{std}(\mathbf{T}_k)}{\overline{T_k}} \tag{22}$$

Furthermore, there also should be an agreement between the interval length estimates $\overline{T_k}$ and the locations of the right interval boundaries, such that

$$Q_{\mathrm{dist},k} = \frac{\left| \left(\overline{P_k} - \overline{P_{k-1}}\right) - \overline{T_k} \right|}{\overline{T_k}} \tag{23}$$

would be minimal. Smaller values of these parameters would indicate a higher consistency of the estimates and thus also a higher confidence. These two parameters can be aggregated into a single confidence heuristic which ranges from 0 to 1, where a value of 1 indicates the highest possible confidence:

$$\overline{Q}_k = \max(\{1 - 5Q_{\mathrm{std},k} - Q_{\mathrm{dist},k},\ 0\}). \tag{24}$$

By applying a fixed threshold $th_Q$ to each $\overline{Q}_k$, unreliable estimates can be excluded from further analysis.

Two different application scenarios have been tested: beat-to-beat heart rate and breath-to-breath respiratory rate estimation. The performance of the improved method has been analyzed on signals obtained by two types of sensors for both application scenarios. For heart beat interval estimation, standard ECG signals and BCG signals obtained from an unobtrusive bed-mounted sensor were used. Respiratory signals were obtained by a nasal flow sensor (thermistor) as well as by the same bed-sensor.

The data used in the analysis was recorded overnight from eight healthy volunteers (eight female and one male with age 32.8 $\pm$ 13.4 years, BMI: 25.9 $\pm$ 3.7) at the Boston Sleep Center, Boston, MA, USA. For each volunteer a full polysomnography was performed of which the lead II ECG, as well as the nasal flow signals, were used in the analysis. A further signal was acquired using a single electromechanical-film (EMFi) sensor (Emfit Ltd, Vaajakoski, Finland; dimensions: 30 x 60, thickness < 1) mounted on the underside of a thin foam overlay which was then placed on top of the mattress of the regular bed. Mechanical deformation of the electromechanical film generates a signal which is proportional to the dynamic force acting along the thickness direction of the sensor. The sensor was positioned where the subjects' thoraxes would usually lie to record cardiac vibrations (ballistocardiogram) and respiratory movements of the person lying in bed.

The performance of the algorithm with respect to the estimated interval lengths has been measured by computing the following error statistics. For each estimated interval, the related interval obtained through a reference method was determined and the relative error between the two was computed. These errors were then aggregated by computing their mean ($\overline{E}$) as well as their 90th percentile ($E_{90}$), which describes the spread of the errors. Furthermore, the coverage denotes the percentage of the reference intervals for which a corresponding interval could be estimated by the improved method.

The automatic detection of R peaks from an ECG is a widely studied and relatively easy task due to the prominence of the R peak, which makes the signal very well suited for peak detecting approaches. The algorithm described above, however, was designed with signals from unobtrusive sensors in mind where such a peak detecting approach would prove too error-prone, such as a ballistocardiogram (BCG).

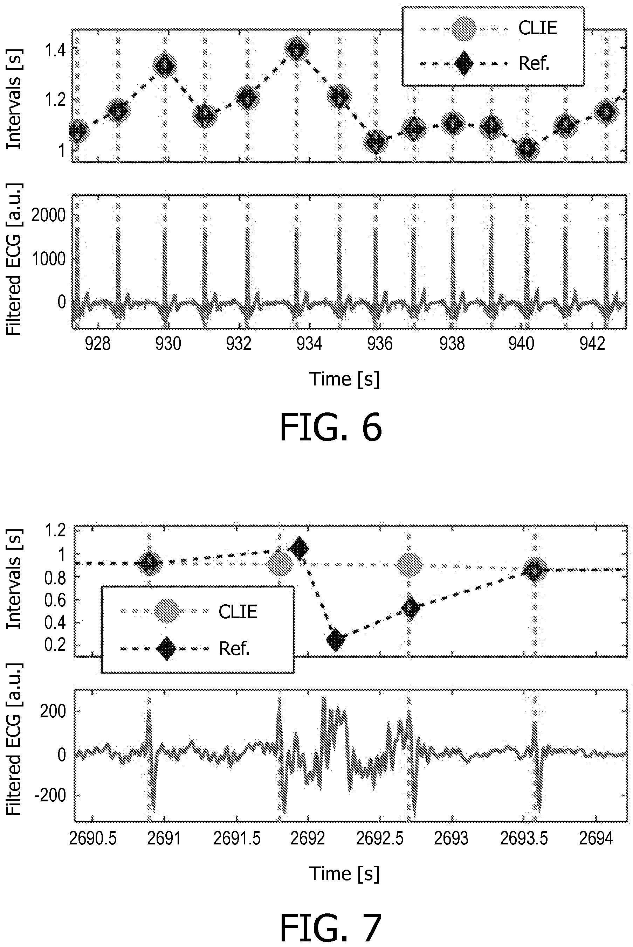

Figure 6 shows RR intervals (top) derived from a clean ECG recording (bottom) by means of the algorithm (CLIE) and the reference algorithm (Ref.). The locations of R peaks in the ECG are marked by vertical dotted lines. This Figure shows a short segment of an ECG recording. As reference, R peaks in the ECG were detected by means of the established Hamilton-Tompkins algorithm. The derived RR intervals are used as reference with the intervals estimated by the algorithm plotted on top. The algorithm is capable of correctly tracking the significant beat-to-beat variations in this example. The grey dots of the algorithm output coincide almost exactly with the black diamonds of the reference signal.

Figure 7 shows RR intervals (top) derived from a noisy ECG recording (bottom) by means of the algorithm (CLIE) and the reference algorithm (Ref.). The locations of R peaks in the ECG are marked by vertical dotted lines. In this case, the signal between two beats is corrupted by noise. The artifact causes the reference algorithm (black diamonds) to incorrectly report an R peak. The algorithm described above (grey dots), however, correctly computes the local intervals since it does not rely on detecting peaks (since it is detecting an interval) and is thus more robust to this type of interference.

Table 1

| Parameter | Heart Rate | Respiration Rate |
|---|---|---|
| Filter pass-band | 0.5-30 Hz | 0-5 Hz |
| $f_s$ | 200 Hz | 10 Hz |
| $\Delta T$ | 0.5 s | 3 s |
| $\Delta t$ | 0.2 s | 1 s |
| $th_Q$ | 0.4 | 0.3 |
| **m** | | $(21\ 1)^T$ |
| $\alpha$ | | 0.1 |
| $\beta$ | | 0.05 |
| $\gamma$ | | 0.01 |
| $\kappa$ | | 2 |

Table 1 shows the algorithm parameters used to process the ECG data. The error statistics for each of the eight overnight recordings (containing more than 212000 heart beats) are shown in the ECG columns of Table 2. Overall, the mean error between the two algorithms with respect to the computed intervals was 0.26 %. Care should be taken when interpreting this result, since the output of the reference algorithm can also deviate from the true underlying intervals (as has been shown in the previous example). Nonetheless, it can clearly be seen that the improved algorithm produces almost identical results (within a small margin of error) to an established reference algorithm.

Table 2

| # | Dur. | Coverage [%] | | $\bar{E}$ [%] | | $E_{90}$ [%] | |
|---|---|---|---|---|---|---|---|
| | (h:mm) | ECG | BCG | ECG | BCG | ECG | BCG |
| 1 | 6:39 | 99.97 | 90.03 | 0.19 | 1.11 | 0.48 | 1.03 |
| 2 | 6:45 | 99.93 | 86.58 | 0.29 | 1.95 | 0.43 | 1.63 |
| 3 | 6:33 | 99.82 | 85.60 | 0.29 | 1.35 | 0.68 | 1.76 |
| 4 | 6:56 | 99.77 | 88.27 | 0.29 | 0.98 | 0.65 | 1.46 |
| 5 | 7:30 | 99.86 | 94.50 | 0.36 | 1.26 | 0.86 | 1.69 |
| 6 | 7:26 | 99.90 | 95.07 | 0.18 | 0.72 | 0.53 | 1.14 |
| 7 | 7:40 | 99.05 | 90.36 | 0.26 | 1.46 | 0.61 | 1.61 |
| 8 | 6:34 | 99.95 | 94.29 | 0.22 | 0.92 | 0.65 | 1.68 |
| **Avg.** | **7:00** | **99.78** | **90.59** | **0.26** | **1.22** | **0.61** | **1.50** |

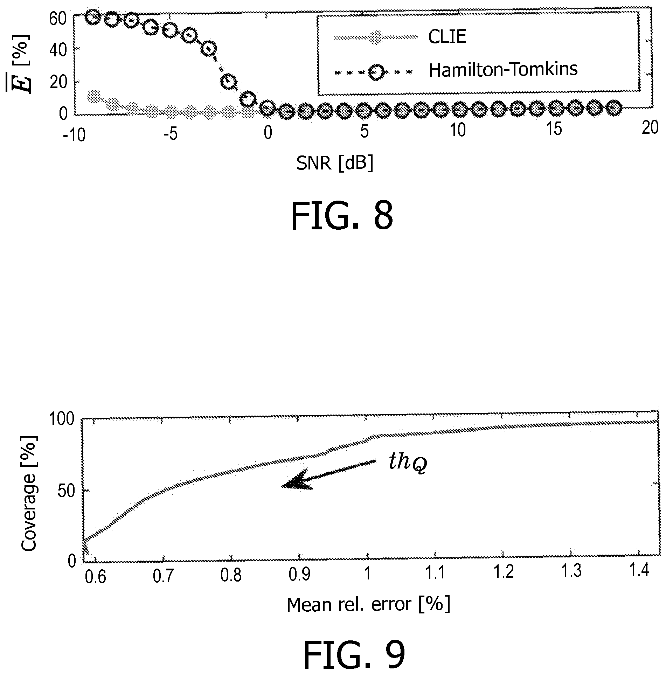

In order to analyze the robustness of the algorithm in the presence of noise, a set of 30-minutes-long ECG samples with different signal to noise ratios (SNRs) was generated by adding white Gaussian noise to a clean base signal. Each of these sample signals were processed by the algorithm as well as by the Hamilton-Tompkins reference algorithm. This was repeated ten times for each SNR. Figure 8 shows the mean relative interval errors ($\overline{E}$) with respect to the reference intervals extracted from the clean ECG base signal. It can be observed that the improved algorithm can maintain acceptable error levels for much lower SNRs than the reference algorithm.

Compared to a standard ECG, the reliable extraction of beat-to-beat heart rates from BCG signals recorded by unobtrusive sensors (such as those integrated into beds) is considerably more challenging. This can mostly be attributed to low signal to noise ratios, high susceptibility to all types of motion artifacts, as well as the fact that they often lack distinctive peaks. BCG signals were obtained from the bed sensor during each of the eight overnight recordings. These BCG signals were analyzed by the improved algorithm using the same parameters that were used during the analysis of the ECG signal in the previous section.

By using a band-pass filter with a high enough cut-off frequency (see Table 1), the low-frequency respiratory components of the bed sensor signals were removed. As before, the ECG RR-intervals obtained by the Hamilton-Tompkins algorithm were used as a gold-standard reference. The BCG columns of Table 2 show the performance of the improved algorithm on this type of signal. As expected, the achieved mean error and coverage values (1.22 % and 90.59 %, respectively) are slightly worse than those achieved when processing the ECG signal. The difference in coverage, however, can be especially attributed to the BCG's higher susceptibility to motion artifacts. When the subject performs major movements in bed, the signal to noise ratio significantly decreases, making a reliable local interval estimation impossible. This is automatically detected by the improved algorithm and such segments are discarded.

Table 3

| Estimator weights | | | Performance | | |
|---|---|---|---|---|---|
| $m_1$ | $m_2$ | $m_3$ | Coverage [%] | $\overline{E}$ [%] | $E_{90}$ [%] |
| 0 | 0 | 1 | 66.64 | 11.90 | 27.16 |
| 0 | 1 | 0 | 85.22 | 21.44 | 54.65 |
| 1 | 0 | 0 | 89.12 | 1.79 | 1.88 |
| 1 | 1 | 1 | 90.14 | 1.33 | 1.69 |
| 2 | 1 | 1 | 90.59 | 1.22 | 1.50 |

Table 3 shows the influence of the three interval estimation methods (P-spectrum, max-spectrum, and cepstrum) on the overall performance under varying weights for the three interval estimators: P-spectrum ($m_1$), max-spectrum ($m_2$), and cepstrum ($m_3$). It can be observed that the P-spectrum method by itself is by far the most accurate and robust estimator. However, by adding the two other methods and giving the P-spectrum twice the weight, it is possible to still further reduce the estimation error.

Figure 9 shows the coverage over mean interval error of the BCG heart rate analysis as a function of the quality threshold $th_Q$. The effect of the quality threshold $th_Q$ to the coverage as well as the interval errors is shown in this Figure. It can be observed that $th_Q$ can be used to adjust the trade-off between coverage and mean errors. Higher values of $th_Q$ exclude more estimates, thus lowering the coverage, but at the same time the average interval error of the remaining estimates is decreased. The arrow in the Figure shows the quality threshold $th_Q$ being decreased from right to left in the Figure.

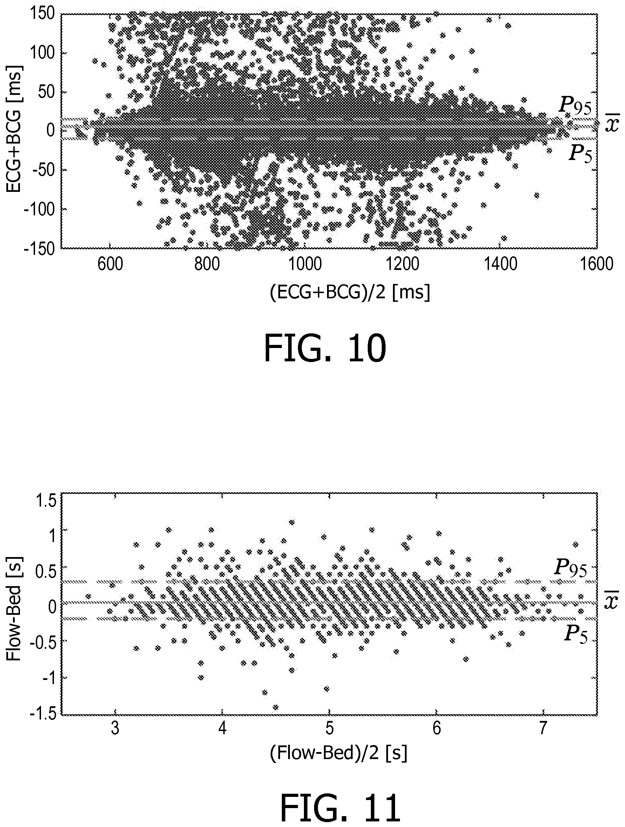

Figure 10 shows a modified Bland-Altman plot of the local interval errors obtained from the BCG signals using the improved algorithm compared to the ECG reference intervals. The plot shows a small bias of 6 ms in the interval errors with 90% of the errors lying between -10 and 15 ms. The equivalent of which is a mean absolute heart rate error of 0.86 beats/min with a 90th percentile of 1.08 beats/min.

A second application for the improved algorithm is the estimation of breath-to-breath intervals. Again, the method was first applied to a reference signal (i.e. a nasal flow signal measured by a thermistor) before moving to a more challenging unobtrusive sensor signal. As reference, peaks in the flow signal were automatically detected using known techniques. Outliers and artifacts were manually excluded from the reference prior to further processing. Reference breath-to-breath intervals were computed as the distances between the detected peaks.

Table 1 above shows the parameters used for processing the respiratory signals using the improved method. The "flow" columns of Table 4 show the performance of the improved algorithm with respect to the reference intervals. The eight analyzed nights contained over 52000 breaths. Overall, a mean relative interval error of 1.72 % with a coverage of 97.25% could be achieved. Table 4 shows the breath-to-breath respiratory rate performance of the improved algorithm when applied to nasal flow and bed sensor (BCG) signals, respectively.

Table 4

| # | Dur. | Coverage (%) | | $\bar{E}$ (%) | | $E_{90}$ (%) | |
|---|---|---|---|---|---|---|---|
| | (h:mm) | Flow | Bed | Flow | Bed | Flow | Bed |
| 1 | 6:39 | 97.00 | 91.93 | 1.48 | 2.36 | 2.94 | 4.76 |
| 2 | 6:45 | 96.55 | 91.85 | 1.96 | 2.94 | 3.75 | 5.26 |
| 3 | 6:33 | 97.27 | 80.94 | 1.93 | 2.76 | 3.70 | 4.84 |
| 4 | 6:56 | 96.18 | 89.48 | 1.96 | 2.15 | 4.17 | 3.70 |
| 5 | 7:30 | 99.47 | 93.52 | 1.22 | 2.36 | 3.12 | 4.69 |
| 6 | 7:26 | 95.51 | 91.15 | 2.16 | 3.29 | 4.88 | 6.25 |
| 7 | 7:40 | 97.36 | 85.43 | 1.81 | 3.40 | 3.57 | 6.45 |
| 8 | 6:34 | 98.66 | 94.56 | 1.27 | 1.88 | 2.86 | 3.12 |
| **Avg.** | **7:00** | **97.25** | **89.86** | **1.72** | **2.64** | **3.62** | **4.89** |

As an example of an unobtrusively recorded respiratory signal, the bed sensor signal was processed by the improved algorithm, again using the same parameters that were used to process the flow sensor signals. This time, the low-frequency respiratory components in the bed sensor signals were preserved by the pre-processing band-pass filter. The performance statistics on this dataset are shown in the "bed" columns of Table 4. Again, the mean error and coverage values (2.64 % and 89.86 %, respectively) are slightly worse than those obtained from the flow signals. Figure 11 shows a modified Bland-Altman plot of the local interval errors obtained from the bed-sensor breathing signals using the improved algorithm compared to the flow sensor reference intervals. It can be observed that a small bias of 20 ms with 90% of the errors lying between -200 and 300 ms. This corresponds to a mean absolute respiratory rate error of 0.30 breaths/min with a 90th percentile of 0.62 breaths/min.

The flexible online-capable algorithm provides the estimation of local intervals from physiological signals by means of continuous local interval estimation. The improved algorithm preferably combines three methods to obtain robust interval estimates from a variety of signals. Two possible applications of the algorithm (beat-to-beat heart rate and breath-to-breath respiratory rate estimation) are validated using a classical and an unobtrusive sensor signal for each application. Based on an evaluation data set of eight overnight sleep-lab recordings, the algorithm's performance was compared to established reference algorithms working on reference signals. Analyzing signals from an unobtrusive bed-sensor, the improved algorithm achieved a mean beat-to-beat heart rate interval error of 1.22% with a mean coverage of 90.58%. Using the same signals to estimate breath-to-breath respiratory rates resulted in a mean error of 2.64% and in a coverage of 89.86%.

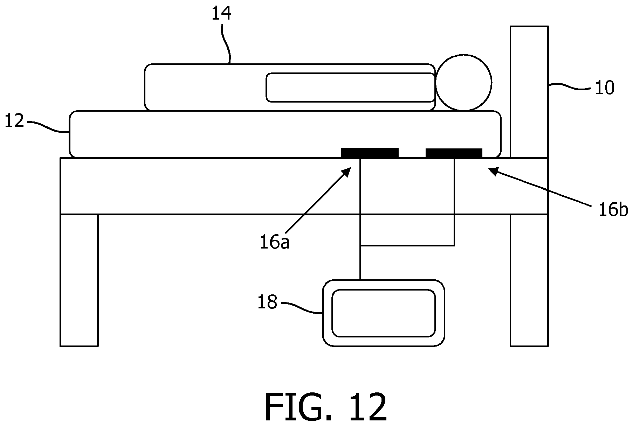

Figure 12 shows a second embodiment of the system that can use the improved algorithm discussed above. Figure 12 is very similar to Figure 1, except that two sensors 16a and 16b are used, rather than a single sensor 16, as shown in Figure 1. The two sensors 16a and 16b need not necessarily be the same kind of sensor, but they must be designed to sense the same physiological rhythm of the subject 14. The second sensor 16b could be body contacting sensor, for example, rather than a second, under-mattress sensor, as shown here. If measuring heart rate for example, one sensor could be a BCG sensor and the other sensor could be an ECG sensor.

The two signals produced by the two sensors 16a and 16b can be kept separate and each processed individually according to the algorithm discussed above. Multiple overlapping analysis windows will be used on each individual signal with multiple interval length estimation methods being used in each analysis window in order to determine an interval length for each signal. These determined interval lengths can then be combined into a single reliable estimation of the interval length for the physiological rhythm that is being monitored by the system of Figure 12. In this way, a more accurate estimation of the interval present within the monitored physiological rhythm of the subject 14 is made.

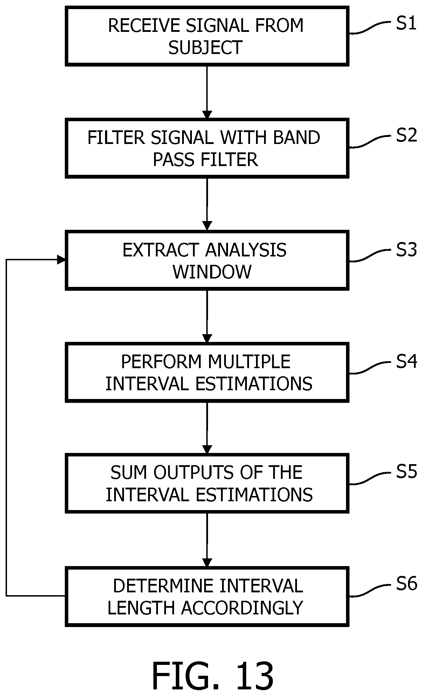

The improved method of processing the signal (which is representing a physiological rhythm of the subject) is summarized in Figure 13. In its most basic form, the method comprises the steps of, step S1, receiving the signal from the subject, step S2, filtering the signal with a band pass filter, step S3, extracting an analysis window from the filtered signal, step S4, performing a plurality of interval length estimation methods on the filtered signal in the analysis window, step S5, summing the outputs of the plurality of interval length estimation methods, and finally step S6, determining an interval length from the sum of the outputs of the plurality of interval length estimation methods. The return arrow from step S6 to step S3 indicates the preferred embodiment which involves moving the analysis window along slightly and repeating the steps S4 to S6 for the new analysis window.

While the invention has been illustrated and described in detail in the drawings and foregoing description, such illustration and description are to be considered illustrative or exemplary and not restrictive; the invention is defined in the appended claims.

**Claims**

1. A computer-implemented method of processing a signal representing a physiological rhythm of a subject (14), the method comprising the steps of:

   a) receiving the signal from the subject,
   b) filtering the signal with a band pass filter,
   c) extracting multiple analysis windows from the filtered signal, the multiple analysis windows comprising at least three analysis windows overlapping each other;
   d) performing a plurality of interval length estimation methods on the filtered signal in each of the multiple analysis windows,
   e) summing the outputs of the plurality of interval length estimation methods,
   f) determining an interval length of the physiological rhythm from the sum of the outputs of the plurality of interval length estimation methods,
   g) detecting a peak in the filtered signal in consecutive analysis windows corresponding to an end of the determined interval length, and
   h) calculating a median determined interval length from the set of matching peaks of the same amplitude.

2. A method according to claim 1, wherein the overlap of a first analysis window and a second analysis window is greater than 50% of the size of the first and/or second analysis windows.

3. A method according to claim 1 or 2, and further comprising calculating a mean interval length for multiple analysis windows, generating upper and lower threshold limits for the interval length from the calculated mean interval length and a predetermined delta and discarding any determined interval lengths that are outside the generated upper and lower threshold limits.

4. A method according to any preceding claim, and further comprising accessing weighting factors for the plurality of interval length estimation methods and wherein the step e) comprises summing the outputs of the plurality of interval length estimation methods according to the accessed weighting factors.

5. A method according to any preceding claim, and further comprising performing a quality analysis of a set of determined interval lengths using a function of the standard deviation of the set and a predetermined threshold.

6. A method according to any preceding claim, and further comprising receiving a second signal from the subject, the second signal representing the same physiological rhythm of the subject as the first signal, processing the second signal according to the steps b) to e) and wherein step f) comprises determining an interval length from the sum of the outputs of the plurality of interval length estimation methods on both signals.

7. A system for processing a signal representing a physiological rhythm of a subject comprising a sensor (16, 16a) arranged to receive the signal from the subject and a processor (18) arranged to perform the method steps of any one of claims 1 to 6.

8. A system according to claim 7, and further comprising a second sensor (16b) arranged to receive a second signal from the subject, the second signal representing the same physiological rhythm of the subject as the first signal, wherein the processor (18) is arranged to process the second signal according to the steps b) to e) and to determine an interval length from the sum of the outputs of the plurality of interval length estimation methods on both signals.

9. A computer program product stored on a computer readable medium for processing a signal representing a physiological rhythm of a subject, the product comprising instructions for performing the method steps of any one of claims 1 to 6.

**Patentansprüche**

1. Computerimplementiertes Verfahren zur Verarbeitung eines Signals, das einen physiologischen Rhythmus eines Subjekts (14) darstellt, wobei das Verfahren die Schritte umfasst:

   a) Empfangen eines Signals von dem Subjekt,

EP 2 819 571 B1

b) Filtern des Signals mit einem Bandpassfilter,

c) Extrahieren mehrerer Analysefenster aus dem gefilterten Signal, wobei die mehreren Analysefenster mindestens drei Analysefenster umfassen, die einander überlappen;

d) Durchführen einer Vielzahl von Intervalllängen-Schätzungsverfahren an dem gefilterten Signal in jedem von den mehreren Analysefenstern,

e) Summieren der Ergebnisse der mehreren Intervalllängen-Schätzungsverfahren,

f) Bestimmen einer Intervalllänge des physiologischen Rhythmus aus der Summe der Ergebnisse der mehreren Intervalllängen-Schätzungsverfahren,

g) Erfassen eines Spitzenwerts in dem gefilterten Signal, der einem Ende der bestimmten Intervalllänge entspricht, in aufeinanderfolgenden Analysefenstern, und

h) Berechnen einer durch Mittelung bestimmten Intervalllänge aus dem Satz übereinstimmender Spitzenwerte der gleichen Amplitude.

2. Verfahren nach Anspruch 1, wobei die Überlappung eines ersten Analysefensters und eines zweiten Analysefensters größer ist als 50 % der Größe des ersten und/oder des zweiten Analysefensters.

3. Verfahren nach Anspruch 1 oder 2, und ferner das Berechnen einer mittleren Intervalllänge für mehrere Analysefenster, das Erzeugen einer oberen und einer unteren Schwellenwertgrenze für die Intervalllänge aus der berechneten mittleren Intervalllänge und einem vorgegebenen Delta und das Verwerfen etwaiger außerhalb der erzeugten oberen und unteren Schwellenwertgrenzen liegenden bestimmten Intervalllängen umfassend.

4. Verfahren nach einem der vorangehenden Ansprüche, und ferner das Abrufen von Gewichtungsfaktoren für die Mehrzahl von Intervalllängen-Schätzungsverfahren umfassend, und wobei der Schritt e) das Summieren der Ergebnisse der Vielzahl von Intervalllängen-Schätzungsverfahren gemäß den abgerufenen Gewichtungsfaktoren umfasst.

5. Verfahren nach einem der vorangehenden Ansprüche, und ferner das Durchführen einer Qualitätsanalyse eines Satzes von bestimmten Intervalllängen unter Verwendung einer Funktion der Standardabweichung des Satzes und eines vorgegebenen Schwellenwerts umfassend.

6. Verfahren nach einem der vorangehenden Ansprüche, und ferner das Empfangen eines zweiten Signals von dem Subjekt, wobei das zweite Signal den gleichen physiologischen Rhythmus des Subjekts darstellt wie das erste Signal, das Verarbeiten des zweiten Signals gemäß den Schritten b) bis e) umfassend, und wobei Schritt f) das Bestimmen einer Intervalllänge aus der Summe der Ergebnisse der Vielzahl von Intervalllängen-Schätzungsverfahren an beiden Signalen umfasst.

7. System zum Verarbeiten eines Signals, das einen physiologischen Rhythmus eines Subjekts darstellt, einen Sensor (16, 16a), der dafür ausgelegt ist, das Signal von dem Subjekt zu empfangen, und einen Prozessor (18), der dafür ausgelegt ist, die Verfahrensschritte nach einem der Ansprüche 1 bis 6 durchzuführen, umfassend.

8. System nach Anspruch 7, und ferner einen zweiten Sensor (16b) umfassend, der dafür ausgelegt ist, ein zweites Signal von dem Subjekt zu empfangen, wobei das zweite Signal den gleichen physiologischen Rhythmus des Subjekts darstellt wie das erste Signal, wobei der Prozessor (18) dafür ausgelegt ist, das zweite Signal gemäß den Schritten b) bis e) zu verarbeiten und eine Intervalllänge aus der Summe der Ergebnisse der Vielzahl von Intervalllängen-Schätzungsverfahren an beiden Signalen zu bestimmen.

9. Computerprogrammprodukt, das auf einem computerlesbaren Medium gespeichert ist, zum Verarbeiten eines Signals, das einen physiologischen Rhythmus eines Subjekts darstellt, wobei das Produkt Befehle zum Durchführen der Verfahrensschritte nach einem der Ansprüche 1 bis 6 umfasst.

**Revendications**

1. Procédé mis en œuvre par ordinateur de traitement d'un signal représentant un rythme physiologique d'un sujet (14), ledit procédé comprenant les étapes suivantes :

   a) la réception du signal du sujet,
   b) le filtrage du signal à l'aide d'un filtre passe-bande,

c) l'extraction de multiples fenêtres d'analyse du signal filtré, les multiples fenêtres d'analyse comprenant au moins trois fenêtres d'analyse se chevauchant ;

d) l'exécution d'une pluralité de procédés d'estimation de la longueur d'intervalle sur le signal filtré dans chacune des multiples fenêtres d'analyse,

e) la sommation des sorties de la pluralité de procédés d'estimation de la longueur d'intervalle,

f) la détermination d'une longueur d'intervalle du rythme physiologique à partir de la somme des sorties de la pluralité de procédés d'estimation de la longueur d'intervalle,

g) la détection d'un pic dans le signal filtré dans des fenêtres d'analyse consécutives correspondant à une fin de la longueur d'intervalle déterminée, et

h) le calcul d'une longueur d'intervalle déterminée médiane à partir de l'ensemble de pics correspondants de même amplitude.

2. Procédé selon la revendication 1, dans lequel le chevauchement d'une première fenêtre d'analyse et d'une deuxième fenêtre d'analyse est supérieur à 50 % de la taille des première et/ou deuxième fenêtres d'analyse.

3. Procédé selon la revendication 1 ou 2, comprenant en outre le calcul d'une longueur d'intervalle moyenne pour de multiples fenêtres d'analyse, la génération des limites de seuil supérieure et inférieure pour la longueur d'intervalle à partir de la longueur d'intervalle moyenne calculée et d'un delta prédéterminé et de rejet de toute longueur d'intervalle déterminée, laquelle est en dehors des limites de seuil supérieure et inférieure générées.

4. Procédé selon l'une quelconque des revendications précédentes, comprenant en outre l'accès à des facteurs de pondération pour la pluralité de procédés d'estimation de longueur d'intervalle et dans lequel l'étape e) comprend la sommation des sorties de la pluralité de procédés d'estimation de la longueur d'intervalle selon les facteurs de pondération accédés.

5. Procédé selon l'une quelconque des revendications précédentes, comprenant en outre l'exécution d'une analyse de qualité d'un ensemble de longueurs d'intervalle déterminées à l'aide d'une fonction de l'écart type de l'ensemble et d'un seuil prédéterminé.

6. Procédé selon l'une quelconque des revendications précédentes, comprenant en outre la réception d'un second signal du sujet, ledit second signal représentant le même rythme physiologique du sujet que le premier signal, le traitement du deuxième signal selon les étapes b) à e) et dans lequel l'étape f) comprend la détermination d'une longueur d'intervalle à partir de la somme des sorties de la pluralité de procédés d'estimation de la longueur d'intervalle sur les deux signaux.

7. Système de traitement d'un signal représentant un rythme physiologique d'un sujet comprenant un capteur (16, 16a) conçu pour recevoir le signal du sujet et un processeur (18) conçu pour mettre en œuvre les étapes du procédé selon l'une quelconque des revendications 1 à 6.

8. Système selon la revendication 7, comprenant en outre un second capteur (16b) conçu pour recevoir un second signal du sujet, ledit second signal représentant le même rythme physiologique du sujet que le premier signal, dans lequel le processeur (18) est conçu pour traiter le second signal selon les étapes b) à e) et pour déterminer une longueur d'intervalle à partir de la somme des sorties de la pluralité de procédés d'estimation de la longueur d'intervalle sur les deux signaux.

9. Produit de programme informatique mémorisé sur un support lisible par ordinateur pour traiter un signal représentant un rythme physiologique d'un sujet, ledit produit comprenant des instructions permettant de mettre en œuvre les étapes du procédé selon l'une quelconque des revendications 1 à 6.

FIG. 1

FIG. 2

FIG. 3

FIG. 4

FIG. 5

FIG. 6

FIG. 7

FIG. 8

FIG. 9

FIG. 10

FIG. 11

FIG. 12

```
┌─────────────────────────┐
│   RECEIVE SIGNAL FROM   │╮── S1
│         SUBJECT         │
└────────────┬────────────┘
             │
             ▼
┌─────────────────────────┐
│  FILTER SIGNAL WITH BAND │╮── S2
│       PASS FILTER        │
└────────────┬────────────┘
             │
             ▼
┌─────────────────────────┐
│     EXTRACT ANALYSIS     │╮── S3
│         WINDOW           │
└────────────┬────────────┘
             │
             ▼
┌─────────────────────────┐
│    PERFORM MULTIPLE      │╮── S4
│   INTERVAL ESTIMATIONS   │
└────────────┬────────────┘
             │
             ▼
┌─────────────────────────┐
│   SUM OUTPUTS OF THE     │╮── S5
│   INTERVAL ESTIMATIONS   │
└────────────┬────────────┘
             │
             ▼
┌─────────────────────────┐
│   DETERMINE INTERVAL     │╮── S6
│   LENGTH ACCORDINGLY     │
└─────────────────────────┘
```

FIG. 13

**EP 2 819 571 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2011025102 A1